(19) Europäisches Patentamt
European Patent Office
Office européen des brevets

(11) Publication number: 0 213 907 B1

# (12) EUROPEAN PATENT SPECIFICATION

(45) Date of publication of the patent specification: 11.01.89

(51) Int. Cl.⁴: C07C 127/22, A01N 47/34

(21) Application number: 86306543.9

(22) Date of filing: 22.08.86

ERRATUM

(SEITE, SPALTE, ZEILE)
(PAGE, COLUMN, LINE)
(PAGE, COLONNE, LIGNE)

DIE TEXTSTELLE :
TEXT PUBLISHED :
LE PASSAGE SUIVANT :

| | | | |
|---|---|---|---|
| Ref. 1 | 1.0 | 100 | 0.01 |
| | 0.5 | 82 | 0.005 |
| | 0.1 | 22 | 0.002 |
| | 0.05 | - | 0.001 |

5 | 22/25

LAUTET BERICHTIGT:
SHOULD READ :
DEVRAIT ETRE LU :

| | | | |
|---|---|---|---|
| Ref. 1 | 1.0 | 100 | 0.01 |
| | 0.5 | 93 | 0.005 |
| | 0.1 | 80 | 0.002 |
| | 0.05 | 61 | 0.001 |

Tag der Entscheidung über die Berichtigung / Date of decision on rectification / Date de décision portant sur modification: 22.03.89

Ausgabe- und Veröffentlichungstag / Issue and publication date / Date d'edition et de publication: 31.05.89

Patbl.Nr / EPB no / Bull. no: 89/22

(19) Europäisches Patentamt
European Patent Office
Office européen des brevets

(11) Publication number: 0 213 907 B1

# (12) EUROPEAN PATENT SPECIFICATION

(45) Date of publication of the patent specification: **11.01.89**

(51) Int. Cl.4: **C07C 127/22**, A01N 47/34

(21) Application number: **86306543.9**

(22) Date of filing: **22.08.86**

(54) **N-(2,6-difluorobenzoyl)-N'-3,5-dichloro-4-[3,3,3-trifluoro-2-chloroprop-1-en-1-yl]phenyl-urea having insecticide activity.**

(30) Priority: **22.08.85 IT 2196785**

(43) Date of publication of application: **11.03.87 Bulletin 87/11**

(45) Publication of the grant of the patent: **11.01.89 Bulletin 89/2**

(84) Designated Contracting States: **AT BE CH DE FR GB LI NL SE**

(56) References cited:
**EP-A- 0 123 302**
**EP-A- 0 159 587**
**EP-A- 0 175 416**
**EP-A- 0 179 022**

(73) Proprietor: **ISTITUTO GUIDO DONEGANI S.p.A., Via Caduti del Lavoro, I-28100 Novara(IT)**

(72) Inventor: **Massardo, Pietro, 5, via Fra Bartolomeo, I-20100 Milan(IT)**
Inventor: **Rama, Franco, 1, via Rodi, I-21052 Busto Arsizio Varese(IT)**
Inventor: **Piccardi, Paolo, 8, via E. De Marchi, I-20125 Milan(IT)**
Inventor: **Caprioli, Vincenzo, 8, Via Loriga, I-28028 S.Martino Siccomario Pavia(IT)**

(74) Representative: **Whalley, Kevin et al, MARKS & CLERK 57/60 Lincoln's Inn Fields, London WC2A 3LS(GB)**

**EP 0 213 907 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an insecticidal compound and compositions containing it.

In our European Patent Appln. 0 123 302 there are disclosed insecticidal compounds derived from 1-benzoyl-3-aryl-urea, among which are the compounds of the following restricted general formula:

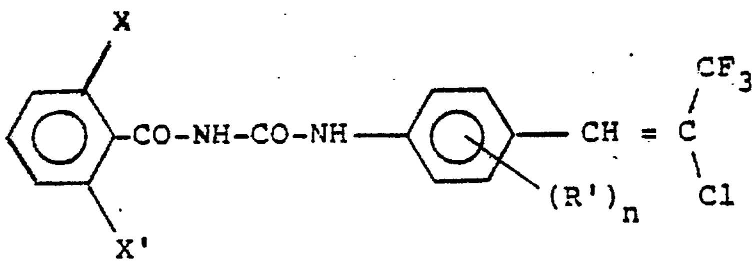

(I)

in which:

one of X and X' is a hydrogen, fluorine or chlorine atom, and the other is a fluorine or chlorine atom;
R' is a hydrogen or halogen atom; and
n is 1 or 2.

During our studies on the compounds disclosed in the EP-A 0 123 302, and in particular on the compounds of formula (I), we have found that, among the compounds of formula (I), the particular compound, N-(2,6-difluorobenzoyl)-N'-3,5-dichloro-4-[3,3,3-trifluoro-2-chloroprop-1-en-1-yl]phenyl-urea, is surprisingly endowed with a decidedly higher insecticidal activity and with a broader spectrum of action.

The invention therefore provides in one embodiment, the compound N-(2,6-difluorobenzoyl)-N'-3,5-dichloro-4-[3,3,3-trifluoro-2-chloroprop-1-en-1-yl]phenyl-urea having the formula:

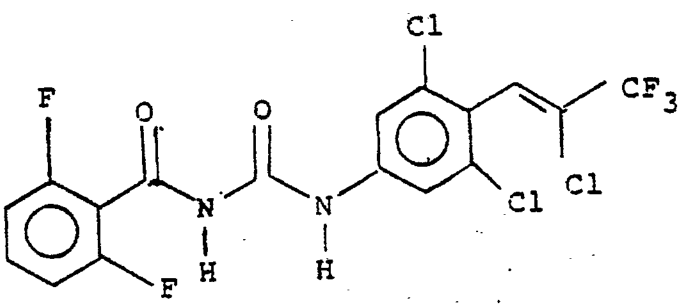

(II)

Compound (II) has a particularly high insecticidal activity which is unexpectedly higher than that of the analogous compounds of formula (I) disclosed in EP-A 0 123 302.

The compound of formula (II) can be used as such, or in the form of suitable compositions, to combat infestations of noxious insects.

The present invention, therefore, further provides the use of compound (II) as an insecticide and insecticidal compositions containing compound (II) as active ingredient.

The preparation of compound (II) is carried out by applying the reaction, already generally described in EP-A 0 123 302, between a benzoyl isocyanate and an aromatic amine, in particular by the reaction between 2,6-difluorobenzoyl isocyanate (III) and 3,5-dichloro-4-[3,3,3-trifluoro-2-chloroprop-1-en-1-yl]aniline (IV) in accordance with the scheme:

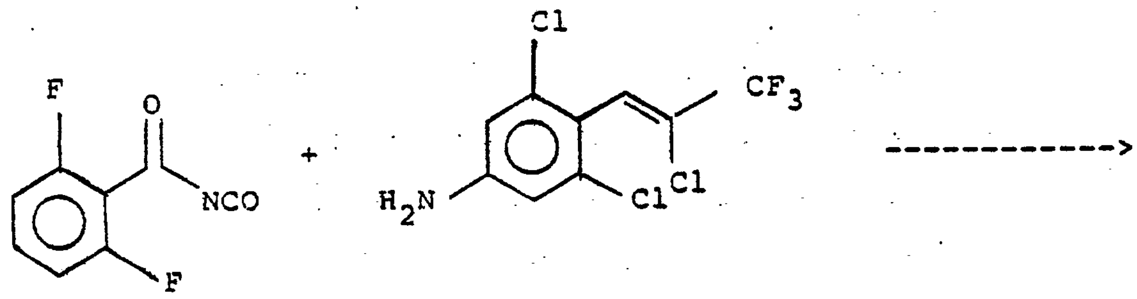

(III) (IV)

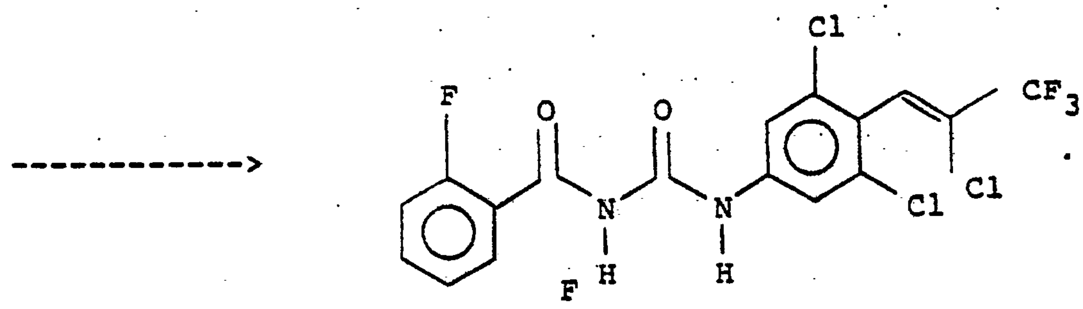

(II)

The reaction does not require the presence of catalysts and is suitably carried out in an inert solvent at a temperature between 0°C and the boiling point temperature of the reaction mixture.

The benzoylisocyanate of formula (III) is a known compound, and can be prepared by methods per se known. The amine of formula (IV) can be prepared by known methods, and more advantageously it can be prepared according to the method as disclosed in Italian Pat. Appln. No. 19080 A/85.

The reaction product can contain small amounts of compound having isomeric configuration at its double bond, which can be separated by the normal chemical-physical routes. However, due to the small difference in activity between the two forms, the purification has not been regarded as necessary.

As mentioned above, N-(2,6-difluorobenzoyl)-N'-3,5-dichloro-4-[3,3,3-trifluoro-2-chloroprop-1-en-1-yl]-phenyl-urea (II) has high insecticidal activity as is particularly active against insect larvae and eggs.

Among these, Compound (II) is especially active against insects belonging to the orders Lepidoptera, Diptera and Coleoptera.

These orders comprise numerous important pests in the agricultural, arboricultural, civil and veterinary fields. Thus, N-(2,6-difluorobenzoyl)-N'-3,5-dichloro-4-[3,3,3-trifluoro-2-chloroprop-1-en-1-yl]-phenyl-urea (II) is adpated for a variety of uses, such as, e.g., the defence of agricultural cultivations against infestations by phytophagous insects, the protection of sites infested by mosquitos and flies, and the protection of breeding-cattle against some cattle parasites.

In practice, N-(2,6-difluorobenzoyl)-N'-3,5-dichloro-4-[3,3,3-trifluoro-2-chloroprop-1-en-1-yl]phenyl-urea (II) can be used as such or, more adequately, in the form of compositions containing, besides the active ingredient, solid or liquid inert carriers and, optionally, further additives. According to the usual formulative practice, the compositions can be supplied as wettable powders, emulsifiable concentrates, etc.

The amount of N-(2,6-difluorobenzoyl)-N'-3,5-dichloro-4-[3,3,3-trifluoro-2-chloroprop-1-en-1-yl]-phenyl-urea (II) in the compositions may vary within a wide range (from 1 to 95% by weight), depending on the type of composition and on the intended use.

Furthermore, the amount of active substance to be used for insecticidal treatments will, of course, depend on such various factors as, the type of infestation, the environment wherein the infestation is occurring (agrarian cultivations, water pools or watercourses, organic substances of various kinds), the type of composition used, climatic and environmental factors, available application means, and so forth. In general, amounts of active substance within the range of from 0.01 to 1 kg/ha are sufficient for a good disinfestation.

In order that the invention may be well understood the following Examples are given by way of illustration only.

Example 1

Preparation of N-(2,6-difluorobenzoyl)-N'-3,5-dichloro-4-[3,3,3-trifluoro-2-chloroprop-1-en-1-yl]-phenyl-urea.

14.0 g of 3,5-dichloro-4-[3,3,3-trifluoro-2-chloroprop-1-en-1-yl]aniline dissolved in 60 ml of anhydrous toluene was introduced, under a nitrogen atmosphere, into a 250-ml three-necked flask equipped with a condenser, dripping funnel and mechanical stirrer.

Then 11.5 g of 2,6-difluorobenzoyl isocyanate dissolved in 40 ml of anhydrous toluene was added dropwise to the flask at room temperature.

The reaction mixture was heated, with stirring, at 100°C for an hour and was then cooled to 0°C. It was then filtered under nitrogen and the precipitate was washed with cold n-hexane and dried under nitrogen.

18 g of benzoyl-urea having a melting point of 180–181°C were obtained.

Example 2

Determination of Insecticidal Activity

Test 1: Activity on larvae of Spodoptera littoralis (Lepidoptera)

Tobacco leaves were treated by mechanical spraying with a solution of N-(2,6-difluorobenzoyl)-N'-3,5-dichloro-4-[3,3,3-trifluoro-2-chloroprop-1-en-1-yl]-phenyl-urea in a water/acetone (10 vol % acetone) mixture containing a surfactant.

After complete evaporation of the solvents, the leaves were infested with second-age larvae of the lepidopteran. The infested leaves were maintained in a suitably conditioned room throughout the test.

By way of comparison, as a control, tobacco leaves which had been treated only with a water/acetone (10 vol % acetone) containing the surfactant, were similarly infested and stored.

Ten days after infestation, and after renewing the treated substrate at least once, the dead larvae were counted, as compared to the control group.

Test 2: Activity on larvae of Aedes aegypti (Diptera)

Spring water (297 ml) was mixed with an acetonic solution (3 ml) of N-(2,6-difluorobenzoyl)-N'-3,5-dichloro-4-[3,3,3-trifluoro-2-chloroprop-1-en-1-yl]-phenyl-urea of suitable concentration.

Into the resulting solution, were introduced 25 4-days-old larvae of the above mentioned dipteran, which were suitably fed. As the control group, other larvae were introduced into a water-acetonic solution (297 ml of spring water, 3 ml of acetone), not containing any active substances.

The number of dead larvae and pupae and of adults normally emerged from the cocoon was noted every 2–3 days, up to the end of the insects emergence from cocoons of the control group.

The activity of the product being tested was expressed as the percent ratio of dead individuals to the total number of treated individuals.

Test 3: Activity on Leptinotarsa decemlineata (Coleoptera)

Potato plants were treated by dipping into a dispersion of N-(2,6-difluorobenzoyl)-N'-3,5-dichloro-4-[3,3,3-trifluoro-2-chloroprop-1-en-1-yl]phenyl-urea in a water/acetone (10 vol % acetone) mixture containing a surfactant. After drying, the plants were infested with groups of 10 second-age larvae of the coleopter, and then suitably stored.

The material under test was preserved throughout the test in a conditioned room under continuous lighting.

After 72–96 hours from the start of treatment, untreated plants were supplied to all the larvae, in order to supply them with fresh feed.

The activity of the product being tested, expressed as the percent death, corrected relatively to the control group, was monitored 8 days after the beginning of the test, by counting the number of the dead larvae, and of those not perfectly vital present in each test group (2 replicates, each constituted by a plant with 10 larvae).

Test 4: Activity on larvae of Heliotis armigera (Lepidoptera)

Tobacco leaves were treated by mechanical spraying with a water/acetone solution of N-(2,6-difluorobenzoyl)-N'-3,5-dichloro-4-[3,3,3-trifluoro-2-chloroprop-1-en-1-yl] phenyl-urea as described in Test 1.

After complete evaporation of the solvents, the leaves were infested with second-age larvae of the lepidopteran. The infested leaves were stood in a suitably conditioned room throughout the test.

By way of comparison, a control group, tobacco leaves treated only with the water/acetone/surfactant solution were similarly infested and stored.

Ten days after the infestation, and after renewing the treated substrate at least once, the dead larvae were counted, as compared to the control group.

In following Table 1, the data relating to the insecticidal activity (% death) at the indicated doses, expressed as parts per million of active substance, of compound (II) of the invention is reported, as compared to that of four analogous compounds, taken as the reference products (Ref. 1, Ref. 2, Ref. 3, Ref. 4), which are described in EP-A 0 123 302.

TABLE 1 – INSECTICIDE ACTIVITY

| | TEST 1 | | TEST 2 | | TEST 3 | | TEST 4 | |
|---|---|---|---|---|---|---|---|---|
| COMPOUND | DOSE, PPM | DEATH | DOSE, PPM | DEATH | DOSE, PPM | DEATH | DOSE, PPM | DEATH |
| (II) | 0.1 | 100 | 0.002 | 100 | 5.0 | 100 | 0.1 | 98 |
| | 0.05 | 95 | 0.0002 | 99 | 1.0 | 92 | 0.05 | 85 |
| | 0.01 | 70 | 0.00002 | 85 | 0.5 | 74 | 0.01 | 61 |
| | 0.005 | 25 | 0.000002 | 40 | 0.1 | 14 | – | – |
| Ref. 1 | 1.0 | 100 | 0.01 | 100 | 50 | 93 | 50 | 64 |
| | 0.5 | 82 | 0.005 | 94 | 10 | 53 | 10 | 22 |
| | 0.1 | 22 | 0.002 | 88 | 5.0 | 50 | 5.0 | 6 |
| | 0.05 | – | 0.001 | 60 | – | – | – | – |
| Ref. 2 | 50 | 100 | 0.2 | 100 | 100 | 100 | – | – |
| | 10 | 82 | 0.02 | 99 | 50 | 97 | – | – |
| | 1.0 | 22 | 0.002 | 50 | 10 | 45 | – | – |
| | – | – | 0.0002 | 0 | 1.0 | 5 | – | – |
| Ref. 3 | 5.0 | 100 | 0.2 | 99 | 100 | 100 | – | – |
| | 1.0 | 37 | 0.02 | 93 | 50 | 97 | – | – |
| | 0.5 | 20 | 0.002 | 88 | 10 | 80 | – | – |
| | – | – | 0.0002 | 17 | 5.0 | 50 | – | – |
| Ref. 4 | 5.0 | 100 | 0.2 | 99 | 50 | 100 | – | – |
| | 1.0 | 93 | 0.02 | 67 | 10 | 47 | – | – |
| | 0.5 | 83 | 0.002 | 45 | – | – | – | – |
| | 0.1 | 27 | 0.0002 | 14 | – | – | – | – |
| | 0.05 | 10 | – | – | – | – | – | – |

Reference 1 is the compound N-(2-chlorobenzoyl)-N'-4-[3,3,3-trifluoro-2-chloroprop-1-en-1-yl]-phenyl-urea; reference 2 is the compound N-(2,6-difluorobenzoyl)-N'-4-[3,3,3-trifluoro-2-chloroprop-1-en-1-yl]-phenyl-urea; reference 3 is the compound N-(2,6-difluorobenzoyl)-N'-3-chloro-4-[3,3,3-trifluoro-2-chloroprop-1-en-1-yl]-phenyl-urea; and reference 4 is the compound N-(2-chlorobenzoyl)-N'-3-chloro-4-[3,3,3-trifluoro-2-chloroprop-1-en-1-yl]-phenyl-urea.

## Claims

1. N-(2,6-Difluorobenzoyl)-N'-3,5-dichloro-4-[3,3,3-trifluoro-2-chloroprop-1-en-1-yl]phenyl-urea.

2. A process for the preparation of the compound N-(2,6-Difluorobenzoyl)-N'-3,5-dichloro-4-[3,3,3-trifluoro-2-chloroprop-1-en-1-yl]phenyl-urea which comprises reacting, in an inert solvent at a temperature of from 0°C to the boiling point of the reaction mixture, N-(2,6-difluorobenzoyl)-isocyanate with 3,5 dichloro-4-[3,3,3-trifluoro-2-chloroprop-1-en-1-yl]aniline.

3. The use of the compound N-(2,6-Difluorobenzoyl)-N'-3,5-dichloro-4-[3,3,3-trifluoro-2-chloroprop-1-en-1-yl]phenyl-urea as an insecticide.

4. An insecticidal composition containing, as active ingredient, the compound N-(2,6-Difluorobenzoyl)-N'-3,5-dichloro-4-[3,3,3-trifluoro-2-chloroprop-1-en-1-yl]phenyl-urea together with a solid or liquid carrier.

5. A method for combating infestations caused by insects, comprising applying to an infested area an effective amount of the compound N-(2,6-Difluorobenzoyl)-N'-3,5-dichloro-4-[3,3,3-trifluoro-2-chloroprop-1-en-1-yl]phenyl-urea, either as such or in the form of a suitable somposition.

**Patentansprüche**

1. N-(2,6-Difluorbenzoyl)-N'-3,5-dichlor-4-[3,3,3-trifluor-2-chlorprop-1-en-1-yl]phenyl-harnstoff.

2. Verfahren zur Herstellung der Verbindung N-(2,6-Difluorbenzoyl)-N'-3,5-dichlor-4-[3,3,3-trifluor-2-chlorprop-1-en-1-yl]phenyl-harnstoff, welches umfasst die Umsetzung, in einem inerten Lösungsmittel bei einer Temperatur von 0° bis zum Siedepunkt der Reaktionsmischung, von N-(2,6-Difluorbenzoyl)-isocyanat mit 3,5-Dichlor-4-[3,3,3-trifluor-2-chloroprop-1-en-1-yl]anilin.

3. Verwendung der Verbindung N-(2,6-Difluorbenzoyl)-N'-3,5-dichlor-4-[3,3,3-trifluor-2-chlorprop-1-en-1-yl]phenyl-harnstoff als Insektizid.

4. Insektizide Zusammensetzung, enthaltend als aktiven Bestandteil die Verbindung N-(2,6-Difluorbenzoyl)-N'-3,5-dichlor-4-[3,3,3-trifluor-2-chlorprop-1en-1-yl]phenyl-harnstoff zusammen mit einem festen oder flüssigen Trägerstoff.

Verfahren zur Bekämpfung von durch Insekten verursachtem Befall, umfassend das Auftragen einer wirksamen Menge der Verbindung N-(2,6-Difluorbenzyl)-N'-3,5-dichlor-4-[3,3,3-trifluor-2-chlorprop-1-en-1-yl]phenyl-harnstoff, entweder als solche oder in der Form einer geeigneten Zusammensetzung, auf eine befallene Fläche.

**Revendications**

1. N-(2,6-difluorobenzoyl)-N'-3,5-dichloro-4-[3,3,3-trifluoro-2-chloroprop-1-en-1-yl]phenyl-urée.

2. Un procédé de préparation du composé consistant en N-(2,6-difluorobenzoyl)-N'3,5-dichloro-4-[3,3,3-trifluoro-2-chloroprop-1-en-1-yl]phenyl-urée qui comprend la réaction, dans un solvant inerte, à une température comprise entre 0°C et le point d'ébullition du mélange réactionnel, de N-(2,6-difluorobenzoyl)-isocyanate avec la 3,5-dichloro-4-[3,3,3-trifluoro-2-chloroprop-1-en-1-yl]aniline.

3. L'utilisation du composé consistant en N-(2,6-difluorobenzoyl)-N'-3,5-dichloro-4-[3,3,3-trifluoro-2-chloroprop-1-en-1-yl]phenyl-urée en tant qu'insecticide.

4. Une composition insecticide contenant en tant qu'agent actif, le composé consistant en N-(2,6-difluorobenzoyl)-N'-3,5-dichloro-4-[3,3,3-trifluoro-2-chloroprop-1-en-1-yl]phenyl-urée associée à un support solide ou liquide.

5. Un procédé pour combattre les infestations dues aux insectes consistant à appliquer aux zones infestées une quantité efficace d'un composé consistant en N-(2,6-difluorobenzoyl)-N'-3,5-dichloro-4-[3,3,3-trifluoro-2-chloroprop-1-en-1-yl]phenyl-urée, tel quel ou sous la forme d'une composition appropriée.